Europäisches Patentamt

European Patent Office

Office européen des brevets

(19)

(11) Publication number: **0 181 609**
**B1**

# EUROPEAN PATENT SPECIFICATION

(12)

(45) Date of publication of patent specification: **28.02.90**

(21) Application number: **85114176.2**

(22) Date of filing: **07.11.85**

(51) Int. Cl.⁵: **C 12 N 1/12,** A 01 K 61/00 //
C12N13/00

(54) Algae cultivating device.

(30) Priority: **07.11.84 JP 234438/84**

(43) Date of publication of application:
**21.05.86 Bulletin 86/21**

(45) Publication of the grant of the patent:
**28.02.90 Bulletin 90/09**

(84) Designated Contracting States:
**CH DE FR GB IT LI NL SE**

(56) References cited:
**EP-A-0 085 926**
**US-A-3 955 317**

(73) Proprietor: **Mori, Kei**
**3-16-3-501, Kaminoge**
**Setagaya-ku Tokyo (JP)**

(72) Inventor: **Mori, Kei**
**3-16-3-501, Kaminoge**
**Setagaya-ku Tokyo (JP)**

(74) Representative: **Patentanwälte Grünecker,**
**Kinkeldey, Stockmair & Partner**
**Maximilianstrasse 58**
**D-8000 München 22 (DE)**

Courier Press, Leamington Spa, England.

## Description

The present invention relates to a concentrated fish breeding device according to the generic part of claim 1.

The present applicant has previously proposed various ways to focus solar rays or artificial light rays by the use of lenses or the like to guide the same into an optical conductor cable and thereby to transmit them onto an optional desired place. The solar rays or the artificial light rays transmitted and emitted in such a way are employed for photo-synthesis and for use in illuminating or for other like purposes, for example, to promote the cultivation of plants or to cultivate algae or the like.

Furthermore, the present applicant has already proposed various culture devices for cultivating algae or the like, for instance, chlorella. Basically, in order to cultivate chlorella, light rays and carbon dioxide $CO_2$ are needed for performing photo-synthesis. By supplying light rays and carbon dioxide $CO_2$ to a chlorella cultivating tub, chlorella is cultivated, and oxygen $O_2$ is created at the same time.

Such a culture device for cultivating chlorella and the like is disclosed in the EP—A—0 085 926. In this device the solar rays or the artificial light rays are focused by use of lenses or the like and guided into an optical conductor and further guided into a chlorella cultivating tub. Those light rays are radiated from the optical conductor into the chlorella cultivating tub. For supplying carbon dioxide $CO_2$ thereto, a commercially prepared carbon dioxide $CO_2$ is supplied to the chlorella.

Another culture device for cultivating algae or the like is disclosed in the US—A—3 955 317. This known device comprises a number of transparent tubes floating on the water surface and partly filled with an aqueous suspension of plant tissue like chlorella. A nutrient medium and carbon dioxide enter the tubes through corresponding liquid and gas inlets and leave the tubes through corresponding liquid and gas outlets. By the combined effect of sunlight passing radially into the transparent tubes and the nutrient medium and the carbon dioxide passing lengthwise through the tubes, the chlorella grows. To harvest the chlorella, the liquid flowing out of the liquid outlets is fed through a separator in which the chlorella are separated from their liquid outputs.

Thus in the prior art devices the chlorella is cultivated separately and away from the fish breeding devices and requires transportation to those devices. Until now it has never been tried to install chlorella culture devices directly in a fish breeding water and feed the cultivated chlorella or the like to the fish directly.

It is an object of the present invention to provide a fish breeding device for effective breeding of fish utilizing chlorella culture devices for cultivating chlorella or the like in an efficient way.

This object and further objects are attained by the characterizing features of claim 1.

The inventive fish breeding device is installed in the natural biotop of the fish to be bred, that is in bodies of water, like the sea, lakes, ponds etc., the natural environmental conditions of which are not affected adversely by the fish breeding device and its operation. The chlorella are cultivated in the fish breeding area and fed to the fish directly without need of any transportation. The structure of the fish breeding device allows to suspend the culture device at any desired place and level within the water below its surface such that fish living in different depths of water are always provided with the required amount of chlorella.

The natural environment, such as the sea, provides the nutrients including the carbon dioxide etc. needed for cultivating the chlorella, which in turn produces oxygen needed by the fish and improving the water quality. The light required for the photo-synthesis by the chlorella, particularly in greater depths of water without sufficient natural lighting, is collected by the solar ray collecting device and transmitted by means of the optical conductor cable to the culture device which is of a very simple construction comprising just a container with an inlet opening and an outlet opening. Thus, both the chlorella and the fish are cultivated and bred respectively in a very efficient way.

Further embodiments of the invention are disclosed in the dependent claims.

The above-mentioned features and other advantages of the present invention will be apparent from the following detailed description which goes with the accompanying drawings.

Brief description of the drawings

Fig. 1 is a perspective construction view for explaining an embodiment of a concentrated fish breeding device according to the present invention; and

Fig. 2 is a construction view for explaining another embodiment of the present invention.

Description of the preferred embodiment

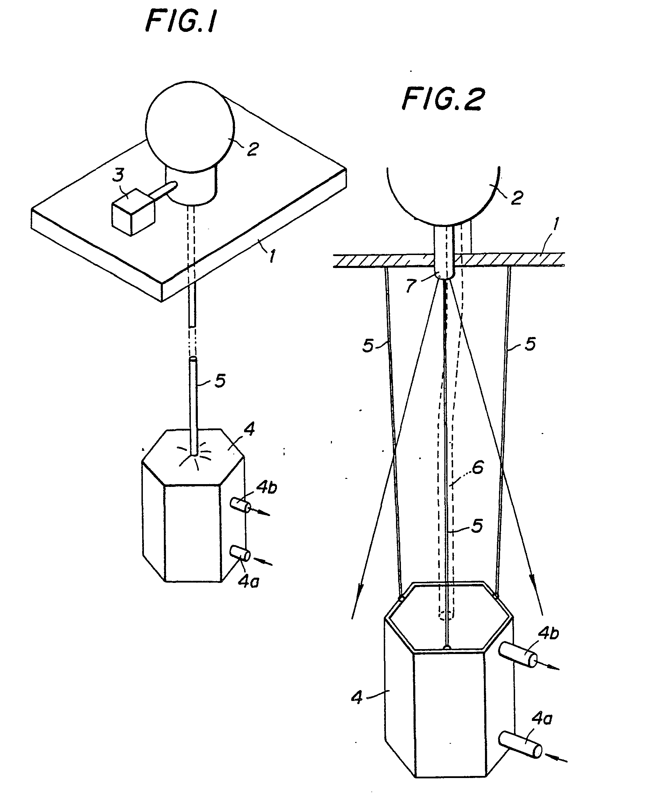

Fig. 1 is a perspective construction view for explaining an embodiment of a concentrated fish breeding device according to the present invention. In Fig. 1, 1 is a floating body in water, 2 a solar ray collecting device installed on the floating body 1, 3 an artificial light source device, including an electric power source such as a generator or a solar battery, 4 a culture device for cultivating the algae or the like such as chlorella, and 5 a wire and/or a cylinder for suspending the chlorella cultivating device 4 from the floating body 1. An optical cable is put along the wire or through the cylinder.

As is well known, the solar ray collecting device 2 focuses solar rays by use of lenses or the like and guides them into the optical conductor cable. The light rays guided into the optical conductor cable in such a manner are supplied to the chlorella cultivating device 4 through the optical conductor cable. In the chlorella cultivating device 4, the light rays are employed as a photo-synthesis light source for cultivating the chlorella.

Furthermore, an artificial light source 3 activated by a solar battery, a storage battery charged by the solar battery and/or a generator are additionally provided. In such a construction, the light rays emitted from the artificial light source are supplied to the chlorella cultivating device through the optical conductor cable. Even when the solar rays are very faint in brightness or cannot be collected at all during the night, the chlorella can still be cultivated in the manner mentioned heretofore.

When the outer circumferential surface of the chlorella cultivating device 4 is constructed with a transparent substance, the light rays employed for cultivating the chlorella will leak from the chlorella cultivating device. Therefore, it not only will gather fish seeking the light rays which leak therefrom, but also the algae will stick the outer surface of the chlorella cultivating device seeking the same. Since algae is a bait for fish, the fish will eat the algae and grow. The outer circumferential surface of the chlorella cultivating tub will also be cleaned in this manner.

Fig. 2 is a construction view of the main portion for explaining another embodiment of the present invention. In Fig. 2, 5 consists of wires for suspending the chlorella cultivating device 4 from the floating body 1, 6 an optical conductor cable for supplying the light rays emitted from the solar ray collecting device 2 and the artificial light source portion 3 to the chlorella cultivating device 4, and 7 is an optical conductor. When a part of the solar rays or the artificial light rays focused in such a manner as mentioned before are radiated from the upper portion of the chlorella cultivating device through the optical conductor 7 as an illumination light source, a large number of fish are attracted to the device. As a result, fish cultivation can be performed much more effectively.

As is previously proposed by the present applicant in Japanese Patent Application No. 165123/1984, the respective chlorella cultivating devices are comprised of an inlet opening 4a for taking in external water and an outlet opening 4b for discharging the cultivated chlorella and/or the oxygen $O_2$ created by cultivating the chlorella. The carbon dioxide $CO_2$, phosphor, nitrogen, nutritious salt, etc. contained in water are taken into the chlorella cultivating device 4 through the inlet opening 4a in order to cultivate the chlorella, and then the cultivated chlorella and/or the oxygen $O_2$, created by cultivating the chlorella, are discharged back into the water through the outlet opening 4b.

Consequently, it follows that the substance needed for cultivating the chlorella is taken in from the water, the oxygen $O_2$ and the bait needed for breeding the fish are produced in the chlorella cultivating device 4 and discharged back into the sea. Therefore, it may be possible to improve the water's quality and cultivate fish at a lower cost and more effectively.

As is apparent from the foregoing description, according to the present invention, it will be possible to improve the water quality and perform the cultivation of fish more effectively and at the same time, in the sea, in lakes, or in ponds, etc.

## Claims

1. A concentrated fish breeding device, characterized by a culture device (4) for cultivating algae or the like suspended from a body (1) floating in the fish breeding water, the culture device (4) having an inlet opening (4a) for taking in fish breeding water and an outlet opening (4b) for discharging the cultivated algae or the like and oxygen $O_2$ directly into the adjacent fish breeding water containing said culture device, and by a solar ray collecting device (2) and an artificial light source (3) mounted on said floating body and connected with the culture device (4) by means of an optical conductor cable (6) leading to the culture device (4) for transmitting thereto the amount of light rays required for cultivating the algae or the like.

2. A concentrated fish breeding device as defined in claim 1, characterized by an optical conductor (7) ending outside of and near said culture device (4) for illuminating same with part of the light rays from said solar ray collecting device (2) and/or said artificial light source (3).

3. A concentrated fish breeding device as defined in claim 1 or 2, characterized in that the circumferential wall of said cultivating device (4) is constructed with a transparent substance for the passage of light.

## Patentansprüche

1. Konzentrierte Fischbrutanlage, gekennzeichnet durch eine Kultiviervorrichtung (4) zum Kultivieren von Algen oder dergleichen, die an einem Bauteil (1) hängt, das in dem Fischbrutwasser schwimmt, wobei die Kultiviervorrichtung (4) eine Einlaßöffnung (4a) zur Aufnahme von Fischbrutwasser und eine Auslaßöffnung (4b) zur Abgabe der kultivierten Algen oder dergleichen und Sauerstoff $O_2$ direkt in das angrenzende Fischbrutwasser aufweist, das die Kultiviervorrichtung enthält, und durch eine Sonnenstrahlenkolletoreinrichtung (2) und eine künstliche Lichtquelle (3), die auf dem schwimmenden Bauteil befestigt sind und mit der Kultiviervorrichtung (4) mittels eines optischen Leiterkabels (6) verbunden sind, das in die Kultiviervorrichtung (4) führt, um dorthin die Menge Lichtstrahlen zu übertragen, die zum Kultivieren der Algen oder dergleichen erforderlich ist.

2. Konzentrierte Fischbrutanlage nach Anspruch 1, gekennzeichnet durch einen optischen Leiter (7), der außerhalb und nahe der Kultiviervorrichtung (4) endet, um diese mit einem Teil der Lichtstrahlen von der Sonnenstrhalenkollektoreinrichtung (2) und/oder der künstlichen Lichtquelle (3) zu beleuchten.

3. Konzentrierte Fischbrutanlage nach Anspruch 1 oder 2, dadurch gekennzeichnet, daß die Umfanswand der Kultiviervorrichtung (4) aus einem transparenten Material zum Durchlassen von Licht besteht.

**Revendications**

1. Un dispositif concentré de pisciculture, caractérisé par un dispositif (4) de culture destiné à la culture des algues ou similaires suspendu à un corps (1) flottant dans l'eau de pisciculture, le dispositif de culture (4) possédant une ouverture d'admission (4a) pour admettre de l'eau de pisciculture et une ouverture d'évacuation (4b) pour évacuer les algues cultivées ou similaires et l'oxygène $O_2$ directement das l'eau adjacente de pisciculture contenant ledit dispositif de culture, et par un dispositif collecteur (4) de rayons solaires et une source (3) de lumière artificielle montés sur ledit corps flottant et reliés au dispositif de culture (4) au moyen d'un câble conducteur optique (6) conduisant au dispositif (4) de culture pour y transmettre la quantité de rayons lumineux nécessaire à la culture des algues ou similaires.

2. Un dispositif concentré de pisciculture comme défini à la revendication 1, caractérisé par un conducteur optique (7) se terminant à l'extérieur dudit dispositif de culture (4) et au voisinage de celui-ci pour éclairer ce dernier avec une partie des rayons lumineux provenant du dispositif collecteur (2) de rayonnement solaire et/ou de ladite source (3) de lumière artificielle.

3. Un dispositif concentré de pisciculture selon la revendication 1 ou 2, caractérisé en ce que la paroi circonférentielle dudit dispositif de culture (4) est constituée d'une substance transparent destinée au passage de la lumière.

FIG.1

FIG.2